(19)

Europäisches
Patentamt
European
Patent Office
Office européen
des brevets

(11) **EP 2 283 895 A1**

(12) **EUROPEAN PATENT APPLICATION**
published in accordance with Art. 153(4) EPC

(43) Date of publication:
**16.02.2011 Bulletin 2011/07**

(21) Application number: **09742176.2**

(22) Date of filing: **04.05.2009**

(51) Int Cl.:
***A61N 2/02*** (2006.01)   ***A61N 1/40*** (2006.01)

(86) International application number:
**PCT/ES2009/000235**

(87) International publication number:
**WO 2009/135970 (12.11.2009 Gazette 2009/46)**

(84) Designated Contracting States:
**AT BE BG CH CY CZ DE DK EE ES FI FR GB GR
HR HU IE IS IT LI LT LU LV MC MK MT NL NO PL
PT RO SE SI SK TR**
Designated Extension States:
**AL BA RS**

(30) Priority: **08.05.2008 ES 200801327**

(71) Applicant: **Universidad De Zaragoza
50009 Zaragoza (ES)**

(72) Inventors:
• **GOYA, Gerardo, Fabián**
  **E-50009 Zaragoza (ES)**
• **CASSINELLI, Nicolás**
  **E-50009 Zaragoza (ES)**
• **IBARRA GARCIA, Manuel Ricardo**
  **E-50009 Zaragoza (ES)**

(74) Representative: **ABG Patentes, S.L.
Avenida de Burgos 16D
Edificio Euromor
28036 Madrid (ES)**

(54)   **MAGNETIC HYPERTHERMIA APPLICATION DEVICE**

(57)    Magnetic hyperthermia application device (1) to apply magnetic hyperthermia treatment to a sample, **characterized** as such because it includes: an LC resonant tank (3), consisting of a first coil (5) and a first condenser (6), connected to an alternating voltage power supply (2) of variable frequency and a control medium (4) connected to the LC resonant tank (3) and to the alternating voltage power supply (2), which controls the voltage that the alternating voltage power supply (2) applies to the LC resonant tank (3), so that its frequency remains between 99% and 101% of the resonating frequency for this LC tank (3).

FIG. 1

EP 2 283 895 A1

**Description**

**Object of the invention**

The main object of this invention is to serve as a device for the application of magnetic hyperthermia.

**Background of the invention**

Magnetic hyperthermia is a phenomenon of energy absorption in nanostructured ferromagnetic materials, generally magnetic nanoparticles suspended in magnetic colloids (ferrofluids), when subjected to an alternating magnetic field. For historical reasons, the name 'magnetic hyperthermia' is generally given to the superheating of tissues to which ferromagnetic materials have been applied when they are exposed to alternating electromagnetic fields. A growing number of applications for this phenomenon have been proposed in the field of biomedical research.

Microwave hyperthermia is already in use as a complementary therapy to radiotherapy in process for tumoral regression, due to the synergic effects of both types of treatment. The biological basis for hyperthermia therapies is related to the state of hypoxia in the regions affected by tumors that caused reduced sensitivity to radiotherapy and increased sensitivity to hyperthermia in neoplastic cells. To achieve the desired increase in temperature, the process of hyperthermia may involve laser radiation, ionizing radiation and/or microwaves. Although these techniques are able to raise the cell temperature, they present undesired side effects, such as ionization of genetic material or the lack of selectivity.

Hyperthermia with magnetic fluids is of major interest, since it suggests the possibility of highly selective and non-invasive therapies. It consists of the cytolysis of tissues via local hyperthermia through the remote application of an alternating magnetic field to magnetic nanoparticles previously connected to / incorporated in the patient's cells. From the point of view of Physics, hyperthermia with magnetic fluids is related to the dissipation of energy when a ferromagnetic material is subjected to an alternating magnetic field, especially due to magnetic losses (Néel losses) and to the dissipation of energy due to the rotation of nanoparticles (Brown losses).

A device for applying hyperthermia according to the technique above is defined, for example, in Patent Application US 2003/0032995, in which a coil is wrapped around a roughly rectangular, square sectioned magnetic core containing an air gap on one of its sides. When the coil is fed with an alternating current an alternating magnetic flow appears in the core and, therefore, an alternating magnetic field is generated in the air gap, into which the part of the patient's body in need of treatment is introduced.

Another example is described in the document entitled "Cellular level loading and heating of superparamagnetic iron oxide nanoparticles", by Venkat S. Kalambur et al., Longmuir 2007, 23, pages 12329-12336. In this case, the device for applying hyperthermia is not designed for patients, but rather for samples to carry out tests. The device consists exclusively of a coil fed with an alternating current. This creates an alternating longitudinal magnetic field inside the coil, where the sample is introduced.

One of the inconvenient aspects of the devices using the technique described above is that to generate a magnetic field strong enough to produce the effect of hyperthermia in the magnetic particles it is necessary to apply a high alternating current to the inductor or to the coil wrapped around the magnetic core, which requires the use of a large power source. Also, the wiring and other elements in the circuit must be adapted for the flow of high currents. Besides this, the high power of the magnetic field generated means that a major part of the same is left outside the work zone and therefore represents a hazard to the user. In the preceding technique a bulky "cloak" is used to keep the user protected from the magnetic fields.

Another drawback is that the temperature measurement systems used in the preceding technique often fail to produce sufficiently accurate results.

**DESCRIPTION OF THE INVENTION**

This being so, one of the aims of this invention is to provide a magnetic hyperthermia device with a simple operation that is able to generate high current magnetic fields, but the elements of which do not need to withstand high currents.

Another aim of this invention is to improve user safety in relation to exposure to high current magnetic fields.

Other aims of this invention are generally focused on improving magnetic hyperthermia devices.

A hyperthermia device is an instrument capable of generating an alternating magnetic field in a specific zone, prepared to receive a sample, denominated in this document as "work zone". The term "sample" refers to any object to which this hyperthermia treatment is applied, including magnetic solutions for the carrying out of tests, cell culture, animals or parts of the body of a human patient. One can suppose, as known in this technique, that the sample has magnetic properties or has been previously treated with some material that has them.

This being so, in accordance with one aspect of this invention, this description is for a device for applying magnetic hyperthermia to a sample and that comprises:

a) *An LC resonant tank*

This is a coil and a condenser connected in parallel, in a conjoint component denominated in this document as the "LC tank". In this first embodiment, the work zone is located inside the coil of the LC tank. It is well known in this technique that this circuit is capable of generating high currents due to the phenomenon of resonance when an alternating voltage is applied with a frequency close or equal to its resonating frequency. The resonating frequency, by definition, is the frequency at which the reactive components in the impedances on the condenser and the coil cancel themselves. In the LC resonant tank for this invention, the resonating frequency is expressed as follows:

$$f = \frac{1}{2\pi\sqrt{L_{\tan que}C_{\tan que}}}$$

in which $L_{\tan que}$ and $C_{\tan que}$ are respectively the inductance of the coil and the capacity of the condenser.

This being so, when an alternating voltage with a frequency close to its resonating frequency is applied to the LC tank, the reactive components of the coil and the condenser are cancelled, leaving only, to the outside view, their resistance to losses. In this way, at a frequency close to the resonating frequency, the field generated by the LC tank coil, the borne voltage of the LC tank and the current flowing in the tank are maximum, while the current flowing through the LC tank (in other words, through the rest of the circuit), is minimum. This fact offers the advantage that it is possible to feed the coil in the LC tank with high intensities, thereby also generating strong magnetic fields without the need to have an exciter circuit set up to withstand high intensities, thus avoiding the drawbacks relating to the cost and complexity that this implies. In this document the "first condenser" and "first coil" will be denominated respectively as the condenser and the coil comprising the LC tank.

*b) Variable frequency alternating voltage power supply*

This alternating voltage power supply, connected to the LC resonant tank, feeds this latter with an alternating voltage with a frequency close to the resonating frequency of the aforementioned LC tank. In this way, the effect explained above is maximized, in other words, with a low or moderate current flowing to the power supply it is possible to achieve very high intensities inside the LC tank and, therefore, though the coil, thereby obtaining a high strength magnetic field inside the coil. Specifically, in accordance with one of the preferred embodiments for the invention, the frequency of the voltage generated by the variable frequency alternating voltage power supply is between 200 kHz and 1 MHz.

*c) A control means*

The resonating frequency of the LC tank may vary due to changes in the parameters of the first condenser or the first coil, caused by temperature or other conditions. For this reason, the invented device also includes a control means connected to the LC tank and to the alternating voltage power supply, which modifies the frequency of the voltage generated by the alternating voltage power supply to maintain it constantly between 99% and 101% of the resonating frequency of the LC tank.

Besides controlling the frequency, the control means also adjusts the amount of voltage generated by the alternating voltage power supply based on the strength of the magnetic field that the user wishes to apply to the sample and that can be chosen via the use of suitable interfacing media.

Examples of control media include suitably programmed microcontrollers, microprocessors, FPGAs, DSPs or ASICs, among others.

It is possible that for certain applications it is necessary to apply an alternating magnetic field with a different frequency from that found in the LC tank on a specific device for applying hyperthermia. If the resonating frequency of the LC tank is not variable, by modifying the frequency of the voltage from the variable voltage power source to obtain a magnetic field of the desired frequency inside the first coil, the LC tank would no longer function at its resonating frequency and would therefore cease to make use of the main advantage offered by this topology. For this reason, in the most preferred embodiment for the invention, the first condenser for the LC tank has a variable capacity. The modification of capacity C on the first condenser, in accordance with equation shown above, makes it possible to modify the value for the resonating frequency in the LC tank, which in turn makes it possible for the hyperthermia application device to work at the ideal level with different frequencies.

In another preferred embodiment, the variable frequency alternating voltage power supply consists of a direct voltage power supply connected to an inverter. Specifically, in accordance with the most preferred embodiment for the invention, the inverter includes a half bridge circuit, comprising two transistors for which the output is controlled by a pulse generating module and an LC series circuit, consisting of a second coil and a second condenser. The second coil works to raise the voltage, while the second condenser serves to eliminate the zero frequency component, in other words, the direct current component. This being so, in the embodiment, the control means modulates the frequency and amount of pulses generated by the pulse generating module so that the frequency of the output voltage remains constantly similar to the resonating frequency of the LC tank.

In preferred embodiments for the invention, the capacity values for the second condenser and for the inductance of the second coil are, respectively, between 100 nF and 1 $\mu$F and between 10 $\mu$Hy and 200 $\mu$Hy.

Another particular embodiment, the invented device for applying hyperthermia also includes:

*d) At least one component of high magnetic permeability.*

This is a component made of any material with magnetic properties at the operating frequencies, in other words, they must present high magnetic permeability and low magnetic losses at the operating frequencies (200 kHz-1 MHz). Examples of suitable materials are: ferrites doped with zirconium or manganese, or Mu metal (nickel alloy, iron, copper and molybdenum).

With the presence of a component with high magnetic permeability in the device, it is possible to modify the geometry of the magnetic field created by the first coil. By simulating or calculating the geometry of the magnetic field corresponding to each shape and position of the component or components with high magnetic permeability it is possible to design devices suited to different applications. Also, the component or components with high magnetic permeability may serve to "shield" the magnetic field, avoiding the presence outside the hyperthermia application device of excessively high levels that may be potentially harmful to the user. In this way it is possible to reduce the size of the equipment, since the reduction achieved through "shielding" of the field with magnetic materials is much greater than that produced by air, because of the need in this latter case for a bulky "cloak" to keep the user protected from the magnetic field.

This being so, in one particular embodiment of the invention, the component with high magnetic permeability has a roughly rectangular shape in a square or circular section, with an air gap on one side and the first coil wrapped around it. In this case, the work zone into which the sample is introduced is located in the air gap. In another particular embodiment, there are additional components with high magnetic permeability placed around the air gap to shield the magnetic field, so that it is as closely confined within the air gap as possible.

In another particular embodiment, the component with high magnetic permeability is roughly U-shaped and is placed in such a way that the first coil is located "inside" the U along its length. This geometry causes a part of the field to spill out over the imaginary surface that joins the two ends of the U, which is the area constituting the work zone in this embodiment. With a careful design of the geometry and position of the component with high magnetic permeability it is possible to control the overspill of the magnetic field, thereby making it possible to use the device to carry out controlled application of hyperthermia on surfaces, to treat skin lesions or similar, for example. Also, the U-shaped component with high magnetic permeability shields the magnetic field, in such a way that behind the closed end of the U the strength is much lower.

The invented device for the application of magnetic hyperthermia also includes cooling meanss to avoid the heat lost in operation causes overheating in the inductor. For example, the conductor that forms the inductor coil may include an internal duct to circulate cooling water.

Other preferred embodiments for the invented hyperthermia application device include an interfacing means and a communications means.

The interfacing means makes it possible to transmit the data gathered during the hyperthermia treatment to the user, such as data relating to temperatures, treatment time, characteristics of the field applied and/or others.

It also serves for the user to transmit the strength of the magnetic field required or other options to the control means.

The interfacing means may consist of one or a combination of the following elements: an LCD screen, a TFT screen, a touch screen, a group ofLEDs, a keyboard, etc.

The communications means serves to transmit the data acquired during the hyperthermia treatment to a computer or other device. Examples of possible communications media include USB port, Ethernet, Bluetooth, ZigBee etc., as well as communmications media using mobile technology, such as GPRS, UMTS, GSM or others.

In devices based on previous technology, temperature is measured using common instruments, such as thermocouples, inside the sample. However, this procedure for measurement is highly imprecise, especially for certain applications. In order to overcome this disadvantage, one of the embodiments for the device includes an adiabatic Dewar flask placed in the work zone, inside of which the sample is placed along with a means for measuring the temperature. The adiabatic Dewar flask ensures precise measurements regarding the quantity of heat dissipated in the sample during the hyperthermia treatment, using a well known calorimetric expression:

$$Q = m \, C \, \Delta T$$

In which **m** is the sample mass, **C** is the specific heat of the material comprising the sample and $\Delta T = T_f - T_i$ is the difference between the initial and final temperatures during treatment.

Lastly, another particular embodiment for the invention includes a means of introducing the sample that serves to place the sample in the respective work zone in accordance with the specific geometry of the device. Specifically, the means of introducing the sample is via a sliding sample tray.

**DESCRIPTION OF THE DRAWINGS**

To complement the description being made and to aid understanding of the characteristics of the invention, based on an example of practical application of the same, this description includes a set of drawings where, for the purposes of illustration rather than limitation, the following points are represented:

Figure 1.- Shows a preferred embodiment for the invented device, which includes a direct voltage power supply connected to an inverter and an LC tank.

Figure 2.- Shows a detail of the work zone in which the sample is placed in a hyperthermia application device that is not equipped with components with high magnetic permeability.

Figure 3.- Shows a preferred embodiment for a roughly rectangular, square or circular sectioned component with high magnetic permeability.

Figure 4.- Shows a preferred operational mode for a roughly U-shaped component with high magnetic permeability.

**PREFERRED EMBODIMENT OF THE INVENTION**

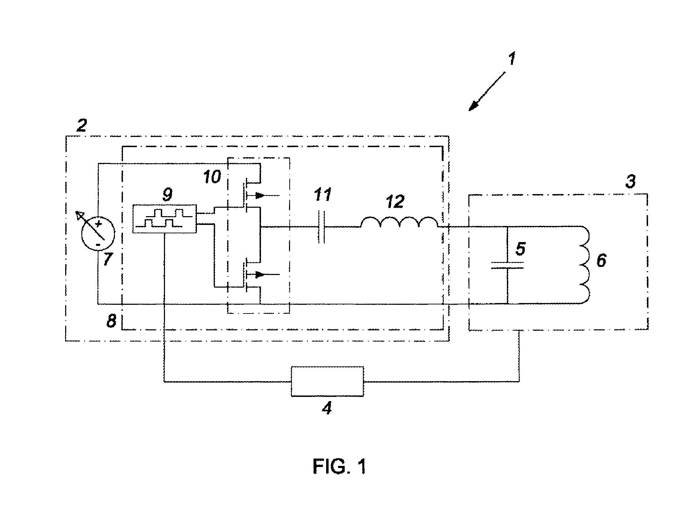

Here we describe the continuation of an example of one of the embodiments for the hyperthermia application device (1) in accordance with the invention. Figure 1 shows an electrical diagram containing an LC tank (3), comprising a first condenser and a first coil (6) for which the capacity and inductance are 100 nF and 4 μHy, respectively. Alternating voltage from an alternating voltage power supply (2) is applied to the LC tank (3), which consists of a direct voltage power supply (7) of variable amplitude between 0 and 300 V connected to an inverter (8). The inverter (8), in turn, comprises a half bridge circuit (10) connected to a pulse generating module (9) and an LC series group, comprised of a second condenser (11) and a second coil(12) with capacity and inductance of 100 nF and 35 μHy, respectively. The half bridge circuit (10), together with the pulse generating module (9) that controls the output of the transistors and the LC series group, transforms the direct voltage generated by the power supply (7) into alternating voltage used to control a control means (4) connected to the pulse generating module (9) and the LC tank (3).

In this way, the control means (4) controls the frequency and voltage of the pulses transmitted to the transistors comprising the half-bridge circuit (10) in order to keep the output voltage of the same as similar as possible to the resonating frequency of the LC tank (3) at each given moment. When the invented device (1) operates at frequencies close to the resonating frequency of the LC tank (3), in this example approximately 251.6 KHz, it is possible to achieve strength of between 0 and 400 A RMS inside the LC tank (3), being that outside the LC tank (3) the strength is approximately between 0 and 20 A RMS.

The control means (4) also controls the amount of direct voltage generated by the power supply (7). In real terms, we know that the magnetic field inside the first coil (6) is proportional to the strength going through it, which in turn is proportional to the voltage applied to the LC tank (3), and that this, in turn, depends on the voltage generated by the direct voltage power supply (7). For this reason, given the needs of the user relating to the strength of the alternating magnetic field, which the user chooses via an interfacing means that is not shown in the drawings, the control means (4) modifies the amount of voltage generated by the direct voltage power supply (7).

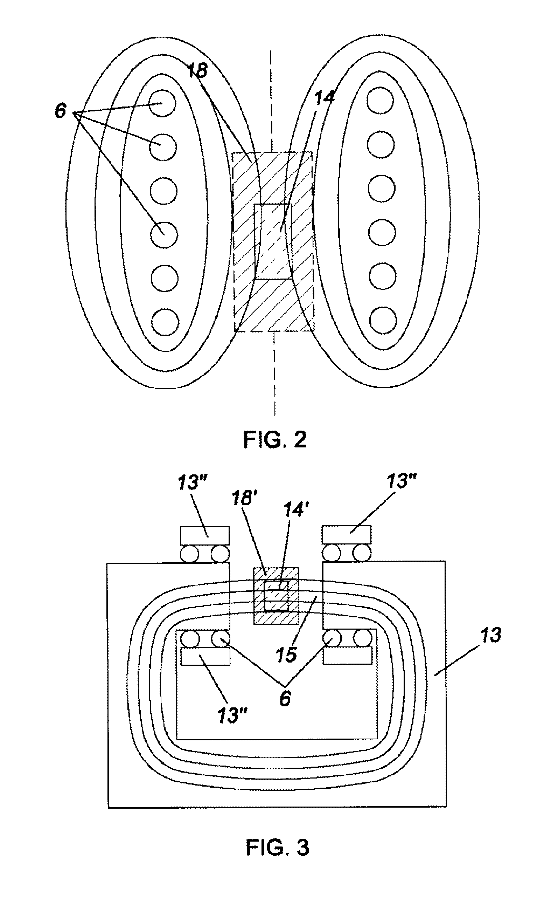

In this way, the invented device (1) makes it possible to modify the magnitude and frequency of the magnetic field generated inside the first coil (6) for each application. In the example shown in Fig. 2, the work zone (18) where the sample is introduced (14) is located inside the first coil (6).

Fig. 3 shows an example in which the first coil (6) for the LC tank (3) is wrapped around a roughly rectangular shaped, square or circular sectioned component with high magnetic permeability (13) and an air gap (15) containing the work zone (18') in which the sample is introduced (14'). It also shows a number of additional components with high magnetic permeability (13"), which serve to shield the user from exposure to the magnetic field.

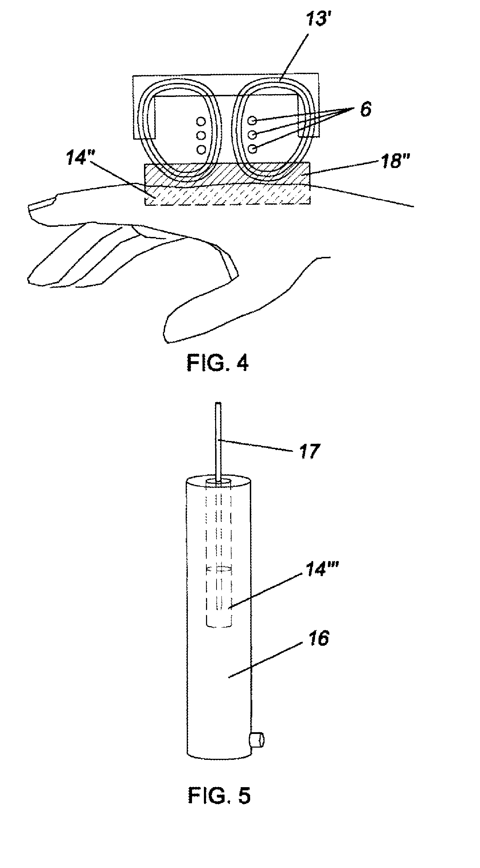

On the other hand, Fig. 4 illustrates a roughly U-shaped component with high magnetic permeability (13'). This geometry causes the field to extend forward, forming a work zone (18") in front of the ends of the U-shape where the sample is located (14"). The magnetic field is insignificant in the area behind the component with high magnetic permeability (13'), thereby minimizing user exposure. This operational mode is useful for applying superficial hyperthermia treatment, such as that used to treat skin infections.

The hyperthermia application device (1) may also include means for measuring the temperature of the sample. Fig. 5 shows an adiabatic Dewar flask (16) which is placed in the work zone (18, 18', 18"), and through the top side of which a optical fiber temperature probe (17) can be introduced, thereby making it possible to obtain precise data regarding the rise in temperature in the sample (14, 14', 14"').

As seen in the figures, the invented device (1) also includes a shell or casing that houses an interfacing means (such as an LCD screen) through which the user can handle the device. It may also include a communications means (such as a USB port) via which the user can download the data for the treatment carried out, such as treatment time, field strength, initial and final temperatures, etc

**Claims**

1.  Magnetic hyperthermia application device (1) for applying magnetic hyperthermia treatment to a sample (14, 14', 14", 14"'), **characterized in that** it comprises

    an LC resonating tank (3), comprising a first coil (6) and a first condenser (5);

an alternating voltage power supply (2) of variable frequency connected to the LC resonating tank (3); and a control means (4), connected to the LC resonating tank (3) and to the alternating voltage power supply (2) of variable frequency, which controls the voltage that the alternating voltage power supply (2) applies to the LC resonating tank (3), so that the frequency remains between 99% and 101% of the resonating frequency of the aforementioned LC tank (3).

2. Magnetic hyperthermia application device (1) in accordance with claim 1, wherein the control means (4) is selected from the following list: a microcontroller, a microprocessor, an FPGA, a DSP and an ASIC.

3. Magnetic hyperthermia application device (1) in accordance with any one of the claims 1, **characterized in that** the first condenser (5) has a variable capacity.

4. Magnetic hyperthermia application device (1) in accordance with claim 1, **characterized in that** the frequency of the alternating voltage applied to the LC resonating tank (3) is between 200 kHz y 1 MHz.

5. Magnetic hyperthermia application device (1) in accordance with claim 1, **characterized in that** the alternating voltage power supply (2) of variable voltage includes a direct voltage power supply (7) connected to an inverter (8).

6. Magnetic hyperthermia application device (1) in accordance with claim 5, **characterized in that** the inverter (8) includes a half bridge circuit (10), comprising two transistors for which the output is controlled by a pulse generating device (9), and an LC series group comprising a second coil (12) and a second condenser (11).

7. Magnetic hyperthermia application device (1) in accordance with claim 6, **characterized in that** the capacity value of the second condenser (11) of the LC series group is between 100 nF and 1 $\mu$F.

8. Magnetic hyperthermia application device (1) in accordance with claim 6, **characterized in that** the inductance value of the second coil (12) of the LC series group is between 10 $\mu$Hy and 200 $\mu$Hy.

9. Magnetic hyperthermia application device (1) in accordance with claim 1, **characterized in that** a work zone (18) is located inside the first coil (6).

10. Magnetic hyperthermia application device (1) in accordance with claims 1-8, **characterized in that** it also includes a part with high magnetic permeability (13, 13', 13") that modifies the geometry of the magnetic field.

11. Magnetic hyperthermia application device (1) in accordance with claim 10, **characterized in that** the square or circular sectioned rectangular part with high magnetic permeability (13), with an air gap (15) on one side, with the first coil (6) wrapped around it.

12. Magnetic hyperthermia application device (1) in accordance with either claim 10 or 11, **characterized in that** the (13") part with high magnetic permeability is placed next to the air gap (15) to shield the magnetic field.

13. Magnetic hyperthermia application device (1) in accordance with any one of the claims 11 or 12, **characterized in that** a work zone (18') is located inside the air gap (15).

14. Magnetic hyperthermia application device (1) in accordance with claim 10, **characterized in that** it includes a U-shaped part with high magnetic permeability (13'), with the first coil (6) located inside along the length of the U.

15. Magnetic hyperthermia application device (1) in accordance with claim 14, **characterized in that** a work zone (18") is located in front of the ends of the U-shaped part with high magnetic permeability (13').

16. Magnetic hyperthermia application device (1) in accordance with claim 1, **characterized in that** it also includes components for cooling the first coil (6).

17. Magnetic hyperthermia application device (1) in accordance with claim 1, **characterized in that** it also includes an adiabatic Dewar flask (16) into which the sample is introduced (14, 14', 14", 14"').

18. Magnetic hyperthermia application device (1) in accordance with claim 1, **characterized in that** it also includes an interfacing means.

19. Magnetic hyperthermia application device (1) in accordance with claim 18, wherein the interfacing means includes at least one of the options from the following list: an LCD screen, a TFT screen, a touch screen, a keyboard and a group of LEDs.

20. Magnetic hyperthermia application device (1) in accordance with claim 1, **characterized in that** it also includes a communications means.

21. Magnetic hyperthermia application device (1) in accordance with claim 20, wherein the communications means includes at least one the options on the following list: USB, Ethernet, Bluetooth, ZigBee, GPRS, UMTS and GSM.

**22.** Magnetic hyperthermia application device (1) in accordance with claim 1, **characterized in that** it also includes a means of introducing a sample (14, 14', 14", 14''') into the work zone (18, 18', 18").

**23.** Magnetic hyperthermia application device (1) in accordance with claim 22, **characterized in that** the means of introducing the sample is a sliding sample tray.

FIG. 1

FIG. 2

FIG. 3

**FIG. 4**

**FIG. 5**

# INTERNATIONAL SEARCH REPORT

| International application No. |
| --- |
| PCT/ ES 2009/000235 |

**A. CLASSIFICATION OF SUBJECT MATTER**

see extra sheet

According to International Patent Classification (IPC) or to both national classification and IPC

**B. FIELDS SEARCHED**

Minimum documentation searched (classification system followed by classification symbols)

A61N

Documentation searched other than minimum documentation to the extent that such documents are included in the fields searched

Electronic data base consulted during the international search (name of data base and, where practicable, search terms used)

INVENES,EPODOC, NPL

**C. DOCUMENTS CONSIDERED TO BE   RELEVANT**

| Category* | Citation of document, with indication, where appropriate, of the relevant passages | Relevant to  claim No. |
| --- | --- | --- |
| X | US 2007179576 A1 (NAGANO et al.) 02.08.2007, description; figures. | 1-23 |
| X | WO 2006092021 A1 (INTERVENTION TECHNOLOGY PTY LT ; WILLIAMS DONALD V) 08.09.2006, pages 7-15; figure 3, figure 7. | 1-8 |
| A | EP 0400940 A2 (LEVEEN HARRY H) 05.12.1990, column 15, lines 4-40; column 17, lines 7-29; column 18, lines 8-22; figures 1-2; figures 11-17. | 10-15 |
| A | US 2005251233 A1 (KANZIUS et al.) 10.11.2005, paragraph [38]; figure 2. | 1-23 |

☐ Further documents are listed in the continuation of Box C.          ☒ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance. | |
| "E" earlier document but published on or after the international filing date | |
| "L" document which may throw doubts on priority claim(s) or which is cited to establish the publication date of another citation or other special reason (as specified) | "X" document of particular relevance; the claimed invention cannot be considered novel or cannot be considered to involve an inventive step when the document is taken alone |
| "O" document referring to an oral disclosure use, exhibition, or other means | "Y" document of particular relevance; the claimed invention cannot be considered to involve an inventive step when the document is combined with one or more other documents , such combination being obvious to a person skilled in the art |
| "P" document published prior to the international filing date but later than the priority date claimed | |
| | "&" document member of the same patent family |

| Date of the actual completion of the international search | Date of mailing of the international search report |
| --- | --- |
| 17 September 2009        (17.09.2009) | **(22/09/2009)** |
| Name and mailing address of the ISA/ O.E.P.M. Paseo de la Castellana, 75 28071 Madrid, España. Facsimile No.   34 91 3495304 | Authorized officer        E. Pina Martínez Telephone No. +34 91 349 85 52 |

Form PCT/ISA/210 (second sheet) (July 2008)

# INTERNATIONAL SEARCH REPORT

Information on patent family members

International application No.

PCT/ ES 2009/000235

| Patent document cited in the search report | Publication date | Patent family member(s) | Publication date |
|---|---|---|---|
| EP 0400940 A | 05.12.1990 | CA 2017816 AC | 01.12.1990 |
| | | EP 19900305782 | 29.05.1990 |
| | | AU 5608390 A | 06.12.1990 |
| | | JP 3094772 A | 19.04.1991 |
| | | US 5099756 A | 31.03.1992 |
| | | AU 643212 B | 11.11.1993 |
| WO 2006092021 A | 08.09.2006 | AU 2006220242 A | 08.09.2006 |
| | | US 2008262490 A | 23.10.2008 |
| US 2005251233 A | 10.11.2005 | US 2005251234 A | 10.11.2005 |
| | | WO 2005110261 A | 24.11.2005 |
| | | CA 2562625 A | 24.11.2005 |
| | | WO 2005110544 A | 24.11.2005 |
| | | US 2005273143 A | 08.12.2005 |
| | | WO 2005118065 A | 15.12.2005 |
| | | WO 2005120639 A | 22.12.2005 |
| | | US 2006190063 A | 24.08.2006 |
| | | US 7510555 B | 31.03.2009 |
| | | EP 1758648 A | 07.03.2007 |
| | | EP 20050779106 | 09.05.2005 |
| | | US 2007250139 A | 25.10.2007 |
| | | JP 2007536016 T | 13.12.2007 |
| US 2007179576 A | 02.08.2007 | WO 2005102452 A | 03.11.2005 |

Form PCT/ISA/210 (patent family annex) (July 2008)

**INTERNATIONAL SEARCH REPORT**

International application No.

PCT/ ES 2009/000235

CLASSIFICATION OF SUBJECT MATTER

*A61N 2/02* (2006.01)
*A61N 1/40* (2006.01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20030032995 A

**Non-patent literature cited in the description**

- **Venkat S. Kalambur et al.** Cellular level loading and heating of superparamagnetic iron oxide nanoparticles. *Longmuir,* 2007, vol. 23, 12329-12336